# EUROPEAN PATENT APPLICATION

(11) **EP 4 420 726 A2**
(43) Date of publication of application: **28.08.2024**
(21) Application number: 24168727.6
(22) Date of filing: 17.03.2016
(51) Int. Cl.: A61P 27/06

(54) **FIXED DOSE COMBINATION OF BROMONIDINE AND TIMOLOL**

(30) Priority: 19.03.2015 US 201562135320 P
(62) Divisional of application: 16712655.6
(71) Applicant: ALLERGAN, INC., Irvine, CA 92612 (US)
(72) Inventor: JIAO, Jim, Irvine, California, 92618 (US); CHANG, Chin-Ming, Tustin, California, 92782 (US); GORE, Anuradha V., Aliso Viejo, California, 92656 (US); GRAHAM, Richard S., Irvine, California, 92612 (US); JORDAN, R. Scott, Trabuco Canyon, California, 92679 (US); NEERVANNAN, Sesha, Irvine, California, 92656 (US); PUJARA, Chetan P., Irvine, California, 92620 (US); SHEN, Jie, Irvine, California, 92620 (US); WARNER, Kevin S., Anaheim, California, 92807 (US)
(74) Representative: Hoffmann Eitle

(57) **Abstract**

Embodiments disclosed herein are directed to fixed compositions comprising brimonidine and timolol for lowering intraocular pressure and treating glaucoma.

## Description

### BACKGROUND OF THE INVENTION

Brimonidine and timolol are medications that have been formulated as ophthalmic solutions known to reduce intraocular pressure ("IOP") in patients with glaucoma or ocular hypertension. The medications are available as monotherapies in various countries in more than one concentration (brimonidine 0.1%, 0.15%, 0.2% and timolol 0.25% and 0.5%). These medications are known to be used concurrently for those with conditions that cannot be adequately controlled by the monotherapies. Formulating brimonidine and timolol into a fixed combination product provides advantages to patients in terms of providing an improvement in benefit-risk, and also simplifying treatment administration.

### BRIEF SUMMARY OF THE INVENTION

In one embodiment, there is provided a composition for the treatment of glaucoma and elevated intraocular pressure, the composition comprising about 0.1% w/v brimonidine tartrate and about 0.68% w/v timolol maleate, the composition being configured for topical ocular administration.

In some embodiments, the composition comprises 0.1% w/v brimonidine tartrate and 0.68% w/v timolol maleate. The composition may comprise 0.1% w/v brimonidine tartrate, 0.68% w/v timolol maleate, 0.1% w/v sodium chloride, 2.15% w/v sodium phosphate dibasic heptahydrate, 0.22% w/v sodium phosphate monobasic monohydrate, and water. The composition may also comprise 0.1% w/v brimonidine tartrate, 0.68% w/v timolol maleate, 0.6% w/v boric acid, 0.38% sodium borate decahydrate, 0.5% w/v carboxymethyl cellulose, 0.32% w/v sodium chloride, and water.

Some compositions may further comprise at least one buffer selected from the group consisting of sodium phosphate dibasic heptahydrate and sodium borate decahydrate. Preferably, the composition has a pH of about 7, or a pH of 7.0. The composition may further comprise one or both of sodium hydroxide and hydrochloric acid.

In some embodiments, administration of the composition twice daily is at least as effective as twice-daily administration of a second composition comprising a fixed dose of 0.2% w/v brimonidine tartrate and 0.5% w/v timolol maleate. In some embodiments, administration of the composition twice daily results in a lower incidence of one or more adverse events as compared to the twice-daily administration of a second composition comprising a fixed dose of 0.2% w/v brimonidine tartrate and 0.68% w/v timolol maleate. The one or more adverse events may be selected from the group consisting of allergic conjunctivitis, conjunctival folliculosis, conjunctival hyperemia, eye pruritus, ocular burning, and ocular stinging. In certain embodiments, the composition does not comprise a preservative. However, in some embodiments, the composition further comprises benzalkonium chloride. The benzalkonium chloride may be present at a concentration of about 0.001% w/v to about 0.05% w/v.

One particular embodiment is a composition for reducing intraocular pressure, the composition consisting essentially of 0.1% w/v brimonidine tartrate, 0.68% w/v timolol maleate, 0.1% w/v sodium chloride, 2.15% w/v sodium phosphate dibasic heptahydrate, 0.22% w/v sodium phosphate monobasic monohydrate, and water.

Another particular embodiment is a composition for reducing intraocular pressure, the composition consisting essentially of 0.1% w/v brimonidine tartrate, 0.68% w/v timolol maleate, 0.6% w/v boric acid, 0.38% sodium borate decahydrate, 0.5% w/v carboxymethyl cellulose, 0.32% w/v sodium chloride, and water.

In another preferred embodiment, there is provided an article of manufacture comprising packaging material and a pharmaceutical agent contained within the packaging material, wherein the packaging material comprises a label which indicates the pharmaceutical agent can be used for lowering intraocular pressure, and wherein the pharmaceutical agent is therapeutically effective for lowering intraocular pressure, the pharmaceutical agent comprising 0.1 % brimonidine tartrate and 0.68% timolol maleate.

In some embodiments, the pharmaceutical agent comprises 0.1% w/v brimonidine tartrate, 0.68% w/v timolol maleate, 0.1% w/v sodium chloride, 2.15% w/v sodium phosphate dibasic heptahydrate, 0.22% w/v sodium phosphate monobasic monohydrate, and water. In some embodiments, the pharmaceutical agent comprises 0.1% w/v brimonidine tartrate, 0.68% w/v timolol maleate, 0.6% w/v boric acid, 0.38% sodium borate decahydrate, 0.5% w/v carboxymethyl cellulose, 0.32% w/v sodium chloride, and water. The pharmaceutical agent may be provided in a unit dose preservative-free configuration. The pharmaceutical agent may be provided in a multi dose preservative-free configuration. The pharmaceutical agent may be provided in a multi dose configuration preserved with one or more preservative agents.

In yet another embodiment, there is provided a method of treating glaucoma or elevated intraocular pressure, wherein the method comprises administering an effective amount of a single composition comprising about 0.1% w/v brimonidine tartrate and about 0.68% w/v timolol maleate.

In some embodiments, the composition is administered twice daily to an eye. In some embodiments, administration of the composition twice daily results in a lower incidence of one or more adverse events as compared to the twice-daily administration of a second single composition comprising a 0.2% w/v brimonidine tartrate and 0.5% w/v timolol maleate. The one or more adverse events may be selected from the group consisting of allergic conjunctivitis, conjunctival folliculosis, conjunctival hyperemia, eye pruritus, ocular burning, and ocular stinging.

### BRIEF DESCRIPTION OF THE DRAWINGS

Figures 1A-C illustrate the results of an experiment measuring ocular tissue and plasma concentrations of brimonidine as a function of time.

Figures 2A-C illustrate the results of an experiment measuring ocular tissue and plasma concentrations of timolol as a function of time.

### DETAILED DESCRIPTION OF THE INVENTION

Embodiments of the present invention relate to the topical ophthalmic use of brimonidine in combination with timolol for treatment of glaucoma, ocular hypertension, and reduction of intraocular pressure. Brimonidine and timolol, two drugs known to lower intraocular pressure ("IOP"), were found to demonstrate synergistic when administered in combination. Among the benefits of such fixed combinations are enhancements in therapeutic efficacy and treatment administration.

Brimonidine is an alpha adrenergic agonist represented by the following formula. The chemical name for brimonidine is 5-Bromo-6-(2-imidazolidinylideneamino) quinoxaline L-tartrate, and its structure is reproduced below.

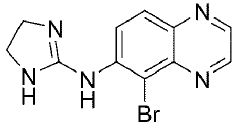

Brimonidine inhibits the activity of adenylate cyclase through the activation of a G protein-coupled receptor. This reduces cAMP and hence aqueous humor production by the iris-ciliary body. The peripheral alpha 2 agonist activity results in vasoconstriction of blood vessels (as opposed to central alpha 2 agonist activity that decreases sympathetic tone, as can be seen by the medication clonidine). This vasoconstriction leads to the acute reduction in aqueous humor flow. An increased prostaglandin release due to alpha adrenergic stimulation from prolonged use of brimonidine increases uveoscleral outflow of aqueous humor through the trabecular meshwork, which in conjunction with brimonidine aqueous humor reduction effect helps lower IOP in treating open angle glaucoma and ocular hypertension.

Brimonidine is commercially available as a monotherapy in several concentrations (0.1%, 0.15%, 0.2% w/v brimonidine tartrate), and is sold by Allergan, Inc. as ALPHAGAN^{®} (or AIPHAGAN^{®} in Japan). While brimonidine is preferably administered as brimonidine tartrate, other salt forms are possible. The free base may be used as well.

Timolol is a non-selective beta-adrenergic receptor antagonist and reduces aqueous humor production through blockage of beta receptors on the ciliary epithelium. But the precise pharmacological mechanism of the ocular hypotensive action of timolol is not clearly established at this time.

The chemical name for timolol is (S)-1-(tert-butylamino)-3-[(4-morpholin-4-yl-1,2,5-thiadiazol-3-yl)oxy]propan-2-ol with the chemical structure shown below.

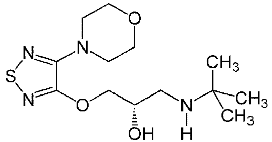

Timolol has been commercially available in several concentrations, including 0.25% and 0.5% w/v timolol. One such brand is TlMOPTOL^{®}. Because timolol is preferably administered using the maleate salt, the preceding concentrations may also be expressed as 0.34% and 0.68% w/v timolol maleate. Of course, timolol may be administered in other salt forms, including the hemihydrate form. The free base may be used as well.

It should be noted that common usage frequently refers to the concentration of timolol in a composition as the free base form without a counter ion. For example, a 0.5% w/v composition of timolol would be typically understood as referring to a 0.5% w/v composition of timolol without a counter ion (i.e., in the free base form). This corresponds to an equivalent concentration of 0.68% w/v timolol maleate.

Conversely, common usage may frequently refer to the concentration of brimonidine in a composition as brimonidine tartrate without explicitly mentioning the tartrate counter ion. For example, a 0.2% w/v composition of brimonidine would typically be understood as referring to a 0.2% w/v composition of brimonidine tartrate, and not brimonidine without a counter ion. The equivalent concentration of brimonidine without a counter ion (i.e., in the free base form) would be 0.132% w/v. Similarly, a 0.1% w/v composition of brimonidine tartrate is equivalent to 0.066% w/v brimonidine free base.

In a monotherapy, 0.2%, 0.15%, or 0.1% brimonidine tartrate may be dosed three times daily, while 0.68% timolol maleate may be dosed twice daily. In certain patients, such as those where monotherapy is insufficient to adequately control intraocular pressure, these two medications may be used at the same time in a concurrent fashion. However, such treatment modalities entail some difficulties, such as having to dose one drug, wait a few minutes, and then dose the second drug. Also, since brimonidine needs to be dosed three times a day, but timolol only twice, this may entail some additional difficulties to the patient in either remembering the single brimonidine dosage or accidentally dosing with timolol. Studies have also shown that adverse events may be increased as a result of such a combined concurrent therapy.

Embodiments of the present invention are directed to a fixed dose composition comprising 0.1% w/v brimonidine tartrate and 0.68% w/v timolol maleate. In a fixed dose composition, brimonidine and timolol are administered in a single composition (e.g., in a single bottle), rather than being administered separately. Such a fixed dose composition provides many benefits as described herein.

Certain fixed dose compositions of brimonidine and timolol, including but not limited to compositions comprising 0.1% w/v brimonidine tartrate and 0.68% w/v timolol maleate, when administered may result in less side effects compared to other drug compositions. For example, administration of the aforementioned compositions may result in less side effects as compared to the administration of a monotherapy comprising 0.2% w/v brimonidine tartrate, or 0.15% w/v brimonidine tartrate, or 0.1% w/v brimonidine tartrate. Further, administration of the aforementioned combinations may result in less side effects as compared to a fixed combination of 0.2% w/v brimonidine tartrate and 0.68% w/v timolol maleate, for example.

Examples of the reduced side effects that may be accredited to the compositions disclosed herein can include, but are not limited to, allergic conjunctivitis, conjunctival folliculosis, conjunctival hyperemia, eye pruritus, ocular burning, and stinging. The compositions disclosed herein may also reduce the incidence of other side effects such as asthenia, blepharitis, corneal erosion, depression, epiphora, eye discharge, eye dryness, eye irritation, eye pain, eyelid edema, eye lid erythema, eyelid pruritus, foreign body sensation, headache, hypertension, oral dryness, somnolence, superficial punctate keratitis, and visual disturbances.

Preferably, the compositions disclosed here are topically administered as a liquid solution, most preferably an aqueous solution. In some embodiments, a liquid emulsion is also possible. Preferably, the compositions described herein are adapted and formulated for topical administration to the eye and ocular surface.

When formulated, certain embodiments may also contain one or more viscosity enhancing agents. Viscosity enhancing agents can include viscosity enhancing polymers capable of increasing viscosity and enhancing mucoadhesion of certain compositions. Without wishing to be bound by theory, the presence of viscosity enhancing agents may prolong the residence time of brimonidine and timolol on corneal surface of the eye, thereby providing better ocular absorption. Certain viscosity enhancing polymers include, among others, xanthan gum, sodium alginate, gellan gum, hyaluronan, pemulan, poloxamer, carbomer, polycarbophil, chitosan, gelatin, pectin, and polyvinylpyrrolidone, polyvinyl alcohol, methyl cellulose, hydroxy propyl methylcellulose, hydroxyethyl cellulose, carboxymethyl cellulose, hydroxylpropyl cellulose, etc. Sodium carboxymethylcellulose is used in Composition B in Table 1 as an example to increase the product viscosity.

Certain embodiments may also provide for one or more additional preservatives. Any preservatives suitable for topical ocular use and compatible with the other compounds present in the composition may be incorporated in the embodiments disclosed herein. These may include, but are not limited to, benzalkonium chloride, thimerosal, chlorobutanol, methyl paraben, propyl paraben, phenylethyl alcohol, edetate disodium, sorbic acid, Purite^{®} (stabilized chlorine dioxide), or other agents known to those skilled in the art. In a preferred embodiment, the preservative is benzalkonium chloride.

The concentration of benzalkonium chloride that may be used may be from about 0.001% w/v to about 0.05% w/v, more preferably from about 0.005% w/v to about 0.02% w/v. Suitable concentrations of thimerosal may be from about 0.001% to about 0.9% w/v. Chlorobutanol may be used from about 0.1% to about 0.5% w/v. Methylparaben may be used from about 0.1% to about 0.3% w/v. Propylparaben may be used from about 0.01% to about 0.2% w/v. Phenylethyl alcohol may be used from about 0.2% to about 0.5% w/v. Ethylenediaminetetraacetic acid (EDTA) may be used from about 0.005% to about 0.2% w/v. Sorbic acid may be used from about 0.05% to about 0.2% w/v. Purite^{®} may be used from about 0.005% to about 0.02% w/v. Appropriate alternative concentrations may of course be used for other preservatives mentioned above.

In certain embodiments, compositions disclosed herein may be prepared as an article of manufacture comprising a pharmaceutical agent with additional packaging material. Such packaging material preferably comprises a label, which may be placed on the exterior of the packaging material, the interior, or as a separate leaflet. This label may comprise information about the composition and pharmaceutical agent contained therein, dosage information, patient safety information, regulatory information, doctor/prescribing information, and the like.

Embodiments disclosed herein may be presented, for example, in a unit dose configuration or a multidose configuration. Typically, a unit dose configuration comprises a single dose of a composition in a container. In some embodiments, this container may be in the form of a non-reusable packaging such as a capsule, LDPE plastic vials, and so forth. Preferably, a unit dose configuration comprises a composition that does not have any added preservative. A multidose configuration may be preserved or preservative-free-that is, the configuration may contain a composition that is preserved or free of preservatives- and typically permits at least two, and preferably multiple, doses to be dispensed from a container containing the multidose configuration. Containers usable in such multidose configurations may comprise typical ophthalmic dropper bottles, or other bottle types. In a multidose preservative-free configuration, to prevent from the risk of ophthalmic infections and possible spoilage of the composition remaining in the container, it is preferable to include some sort of mechanism on the container that will prevent or retard microbial growth. This may include antimicrobial coatings on the container, and container dispensing and venting configurations that prevent microbial entry into the container (e.g., filters, one-way venting, and so on).

### Example 1

Shown in Table 1 are two embodiments of compositions of a fixed dose combination containing 0.1% brimonidine tartrate and 0.5% timolol free base (or 0.68% timolol maleate). These compositions are preferably buffered, isotonic, sterile, and preservative-free ophthalmic solutions for topical administration. The container closure system used for these compositions to deliver the drugs may be unit-dose LDPE plastic vials or advanced multi-dose dropper bottles having self-preserving features to prevent contaminants from ingressing during use. Of course, preserved compositions are also possible.

**Table 1. Examples of Brimonidine and Timolol Fixed Dose Combinations**

| **No.** | **Ingredients** | **Composition A** | **Composition B** |
|---|---|---|---|
| | | **% w/v** | **% w/v** |
| 1 | brimonidine tartrate | 0.1 | 0.1 |
| 2 | timolol maleate | 0.68 | 0.68 |
| 3 | boric acid | N/A | 0.6 |
| 4 | sodium borate decahydrate | N/A | 0.38 |
| 5 | carboxymethylcellulose sodium | N/A | 0.5 |
| 6 | sodium chloride | 0.1 | 0.32 |
| 7 | sodium phosphate dibasic heptahydrate | 2.15 | N/A |
| 8 | sodium phosphate monobasic monohydrate | 0.22 | N/A |
| 9 | 1N sodium hydroxide | Adjust to pH 7.0 | Adjust to pH 7.0 |
| 10 | 1N hydrochloric acid | | |
| 11 | purified water | QS | QS |

Compared to commercially-available brimonidine and timolol monotherapy products, preservative-free embodiments of the Compositions described in Table 1 offer a better safety profile for chronic use of the products in certain patients, such as patients whose ocular surface is susceptible to damage or irritation that may be caused by certain preservatives.

Of course, it should be noted that the embodiments described above are not limited to preservative-free compositions. Any preservatives suitable for topical ocular use can be incorporated in the brimonidine/timolol combination product including, but not limited to, benzalkonium chloride and other preservatives mentioned above. For example, suitable concentrations of benzalkonium chloride may be from about 0.005% to about 0.02% w/v, for example 0.005% w/v, 0.01% w/v, or 0.02% w/v. Suitable concentrations of thimerosal may be from about 0.001% to about 0.9% w/v. Chlorobutanol may be used from about 0.1% to about 0.5% w/v. Methylparaben may be used from about 0.1% to about 0.3% w/v. Propylparaben may be used from about 0.01% to about 0.2% w/v. Phenylethyl alcohol may be used from about 0.2% to about 0.5% w/v. Ethylenediaminetetraacetic acid (EDTA) may be used from about 0.005% to about 0.2% w/v. Sorbic acid may be used from about 0.05% to about 0.2% w/v.

### Example 2

A pharmacokinetic ("PK") experiment was conducted to assess ocular and systemic absorption of brimonidine and timolol in rabbits following topical dosing with the two compositions provided in Table 1.

Table 2 shows the study design for the rabbit experiment with the Table 1 compositions A and B. Rabbits were randomly assigned to groups based on body weight and received a single bilateral topical instillation of the test compositions (Composition A or B) and comparators (Aiphagan^{®} and Timoptol^{®}). Specifically, each group consisted of 12 female rabbits, and two animals (for a total of 4 eyes) were tested at each time point. Dosage consisted of single topical bilateral dosing with a 35 µL dose volume. Samples were collected at 15, 30, 60, 120, 240, 480 minutes post-dose time points and analyzed quantitatively to determine the tissue and plasma concentrations of brimonidine and timolol.

**Table 2. Pharmacokinetics Study of 0.1% Brimonidine tartrate/0.5% Timolol Combo Composition**

| Group | Treatment | PK Collection Timepoints |
|---|---|---|
| 1 | Aiphagan^{®} (0.1% brimonidine tartrate) | 15, 30, 60, 120, 240, 480 minutes post dose |
| 2 | Timoptol^{®} (0.5% timolol) | |
| 3 | Composition A | |
| 4 | Composition B | |
| 5 | Timoptol^{®} followed by Aiphagan^{®a} | 15, 30, 60, 120, 240, 480 minutes post Timoptol dose |
| 6 | Aiphagan^{®} followed by Timoptol^{®a} | |

| | | |
|---|---|---|
| ^{a}: 5 minute interval between dosage of the two eye drops | | |

It was observed that timolol ocular exposure was increased when dosed with the brimonidine tartrate 0.1%/timolol 0.5% combination compositions compared to timolol 0.5% monotherapy. This finding is not obvious to those skilled in the art.

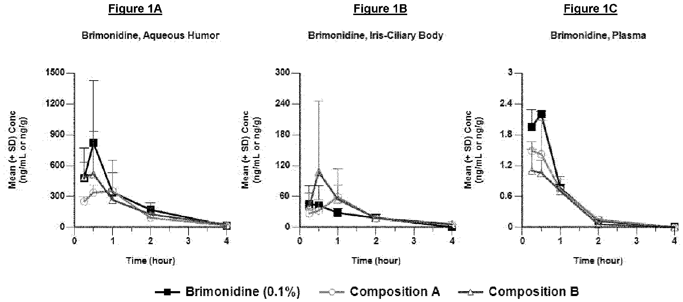

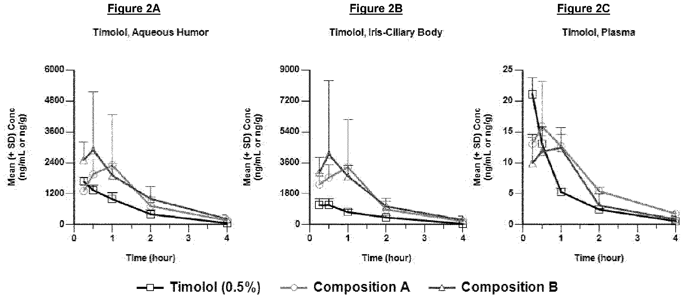

Results of the rabbit PK study are summarized in Tables 3 and 4, as well as Figures 1 and 2. Table 3 and Figure 1 show the amount of brimonidine found in the rabbit aqueous humor, iris-ciliary body, and blood plasma, though it should be noted that the results for the sequential dosing regimens are not reproduced in Figure 1. While the iris-ciliary body is the target tissue for both brimonidine and timolol due to the lOP-lowering effect of these drugs, the aqueous humor is the fluid that bathes the iris-ciliary body. Consequently, drug concentration in the aqueous humor is often highly correlated to the drug concentration in the solid tissues of the iris-ciliary body. Due to naso-lacrimal drainage, topically applied eye drops result in drug exposure in the systemic circulation and the extent of exposure is assessed by drug concentration in the blood plasma, which often correlates to systemic side effects associated with the drugs.

In the foregoing descriptions, "Cₘₐₓ" represents the peak drug concentration reached in the course of the experiment. "AUC" represents the area under the drug concentration-time curve.

Figure 1B shows that, in the iris-ciliary body, both Compositions A and B achieved higher Cₘₐₓ and AUC values for brimonidine compared to the administration of 0.1% brimonidine tartrate alone. This is a promising result because the iris-ciliary body is the target tissue for lowering IOP.

In Figures 1A and 1C, which show aqueous humor and blood plasma brimonidine concentration, respectively, both Compositions A and B resulted in lower Cₘₐₓ and AUC values compared to the administration of 0.1% brimonidine tartrate alone.

Table 3 lists the pharmacokinetic parameters obtained from the experiments illustrated in Figures 1A-C.

In Figures 2A and 2B, both Compositions A and B showed greater ocular exposure to timolol in the aqueous humor and the iris-ciliary body compared to the administration of 0.5% timolol alone. Turning to Figure 2C, blood plasma concentrations of Compositions A and B showed lower concentrations there as compared to the administration of 0.5% timolol alone. These lower plasma concentrations are preferable in order to reduce systemic exposure of timolol, which can have cardiovascular side effects.

Table 4 lists the pharmacokinetic parameters obtained from the experiments illustrated in Figures 2A-C.

**Table 3. Brimonidine PK Profile**

| Matrix | Brimonidine (0.1%) | Composition A | Composition B | Timolol (0.5%) + Brimonidine (0.1%)^{a} | Brimonidine (0.1%) + Timolol (0.5%)^{a} |
|---|---|---|---|---|---|
| Brimonidine: Cₘₐₓ (ng/ml or ng/g) - (Mean ± SE) | | | | | |
| Aqueous Humor | 818 ± 303 | 351 ± 175 | 518 ± 208 | 587 ± 125 | 468 ± 79 |
| Iris-Ciliary Body | 45.1 ± 17.9 | 58.8 ± 27.6 | 108 ± 69 | 68.4 ± 22.2 | 59.9 ± 20.7 |
| Plasma | 2.20 ± 0.04 | 1.49 ± 0.03 | 1.10 ± 0.40 | 1.29 ± 0.58 | 0.944 ± 0.076 |

| Brimonidine: AUC (ng hr/ml or ng hr/g) - (Mean ± SE) | | | | | |
|---|---|---|---|---|---|
| Aqueous Humor | 1000 ± 150 | 645 ± 138 | 782 ± 99 | 700 ± 66 | 646 ± 77 |
| Iris-Ciliary Body | 82.0 ± 11.4 | 94.1 ± 21.2 | 125 ± 29 | 87.8 ± 14.8 | 74.2 ± 12.5 |
| Plasma | 1.95 ± 0.07 | 1.53 ± 0.20 | 1.23 ± 0.19 | 1.32 ± 0.27 | 0.887 ± 0.142 |

| | | | | | |
|---|---|---|---|---|---|
| ^{a}: 5 minute interval between dosage of the two eye drops Cₘₐₓ values are comparable across groups (p>0.05) per one way ANOVA | | | | | |

**Table 4. Timolol PK Profile**

| Matrix | Timolol (0.5%) | Composition A | Composition B | Timolol (0.5%) + Brimonidine (0.1%)^{a} | Brimonidine (0.1%) + Timolol (0.5%)^{a} |
|---|---|---|---|---|---|
| Timolol: Cₘₐₓ (ng/ml or ng/g) - (Mean ± SE) | | | | | |
| Aqueous Humor | 1670 ± 90 | 2300 ± 1140 | 2890 ± 1130 | 2070 ± 380 | 3510 ± 700 |
| Iris-Ciliary Body | 1130 ± 180 | 3330 ± 1400 | 4090 ± 2140 | 2400 ± 380 | 6430 ± 1670 |
| Plasma | 21.1 ± 1.9 | 15.9 ± 5.2 | 12.4 ± 2.3 | 12.3 ± 0.1 | 16.1 ± 1.5 |

| Timolol: AUC (ng hr/ml or ng hr/g) - (Mean ± SE) | | | | | |
|---|---|---|---|---|---|
| Aqueous Humor | 2410 ± 160 | 4330 ± 910 | 5300 ± 630 | 3810 ± 260 | 4490 ± 550 |
| Iris-Ciliary | 1980 ± 130 | 6010 ± 1120 | 6760 ± 980 | 4080 ± 310 | 5570 ± 800 |
| Body | | | | | |
| Plasma | 19.6 ± 1.0 | 32.4 ± 2.4 | 23.5 ± 3.0 | 15.7 ± 0.3 | 21.4 ± 2.7 |

| | | | | | |
|---|---|---|---|---|---|
| ^{a}: 5 minute interval between dosage of the two eye drops Cₘₐₓ values are comparable across groups (p>0.05 per one way ANOVA) | | | | | |

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques.

The terms "a," "an," "the" and similar referents used in the context of describing the invention (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of any claim. No language in the specification should be construed as indicating any non-claimed element essential to the practice of the invention.

Groupings of alternative elements or embodiments disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, the claims include all modifications and equivalents of the subject matter recited in the claims as permitted by applicable law. Moreover, any combination of the above-described elements in all possible variations thereof is contemplated unless otherwise indicated herein or otherwise clearly contradicted by context.

It is to be understood that the embodiments disclosed herein are illustrative of the principles of the claims. Other modifications that may be employed are within the scope of the claims. Thus, by way of example, but not of limitation, alternative embodiments may be utilized in accordance with the teachings herein. Accordingly, the claims are not limited to embodiments precisely as shown and described.

The present disclosure further comprises the following embodiments:
1. A composition comprising about 0.1% w/v brimonidine tartrate and about 0.68% w/v timolol maleate.
2. The composition of item 1, wherein the composition comprises 0.1% w/v brimonidine tartrate and 0.68% w/v timolol maleate.
3. The composition of item 2, wherein the composition comprises 0.1% w/v brimonidine tartrate, 0.68% w/v timolol maleate, 0.1% w/v sodium chloride, 2.15% w/v sodium phosphate dibasic heptahydrate, 0.22% w/v sodium phosphate monobasic monohydrate, and water.
4. The composition of item 2, wherein the composition comprises 0.1% w/v brimonidine tartrate, 0.68% w/v timolol maleate, 0.6% w/v boric acid, 0.38% sodium borate decahydrate, 0.5% w/v carboxymethyl cellulose, 0.32% w/v sodium chloride, and water.
5. The composition of item 1, wherein the composition further comprises at least one buffer selected from the group consisting of sodium phosphate dibasic heptahydrate and sodium borate decahydrate.
6. The composition of any preceding item, wherein the composition has a pH of about 7.
7. The composition of item 6, wherein the composition has a pH of 7.0.
8. The composition of any preceding item, wherein the composition further comprises one or both of sodium hydroxide and hydrochloric acid.
9. The composition of any preceding item, wherein administration of the composition twice daily is at least as effective as twice-daily administration of a second composition comprising a fixed dose of 0.2% w/v brimonidine tartrate and 0.5% w/v timolol maleate.
10.The composition of any preceding item, wherein administration of the composition twice daily results in a lower incidence of one or more adverse events as compared to the twice-daily administration of a second composition comprising a fixed dose of 0.2% w/v brimonidine tartrate and 0.68% w/v timolol maleate.
11.The composition of item 10, wherein the one or more adverse events are selected from the group consisting of allergic conjunctivitis, conjunctival folliculosis, conjunctival hyperemia, eye pruritus, ocular burning, and ocular stinging.
12.The composition of any preceding item, wherein the composition does not comprise a preservative.
13.The composition of any of items 1-11, wherein the composition further comprises benzalkonium chloride.
14.The composition of item 13, wherein the benzalkonium chloride is present at a concentration of about 0.001% w/v to about 0.05% w/v.
15. The composition of any preceding item, wherein the composition is configured for topical ocular administration.
16.The composition of any preceding item, wherein the composition is for the treatment of glaucoma and elevated intraocular pressure.
17.A composition for reducing intraocular pressure, the composition consisting essentially of 0.1% w/v brimonidine tartrate, 0.68% w/v timolol maleate, 0.1% w/v sodium chloride, 2.15% w/v sodium phosphate dibasic heptahydrate, 0.22% w/v sodium phosphate monobasic monohydrate, and water.
18.A composition for reducing intraocular pressure, the composition consisting essentially of 0.1% w/v brimonidine tartrate, 0.68% w/v timolol maleate, 0.6% w/v boric acid, 0.38% sodium borate decahydrate, 0.5% w/v carboxymethyl cellulose, 0.32% w/v sodium chloride, and water.
19.An article of manufacture comprising packaging material and a pharmaceutical agent contained within the packaging material, wherein the packaging material comprises a label which indicates the pharmaceutical agent can be used for lowering intraocular pressure, and wherein the pharmaceutical agent is therapeutically effective for lowering intraocular pressure, the pharmaceutical agent comprising 0.1 % brimonidine tartrate and 0.68% timolol maleate.
20.The article of item 19, wherein the pharmaceutical agent comprises 0.1% w/v brimonidine tartrate, 0.68% w/v timolol maleate, 0.1% w/v sodium chloride, 2.15% w/v sodium phosphate dibasic heptahydrate, 0.22% w/v sodium phosphate monobasic monohydrate, and water.
21.The article of item 19, wherein the pharmaceutical agent comprises 0.1% w/v brimonidine tartrate, 0.68% w/v timolol maleate, 0.6% w/v boric acid, 0.38% sodium borate decahydrate, 0.5% w/v carboxymethyl cellulose, 0.32% w/v sodium chloride, and water.
22.The article of any of items 19-21, wherein the pharmaceutical agent is provided in a unit dose preservative-free configuration.
23.The article of any of items 19-21, wherein the pharmaceutical agent is provided in a multi dose preservative-free configuration.
24.The article of any of items 19-21, wherein the pharmaceutical agent is provided in a multi dose configuration preserved with one or more preservative agents.
25.A method of treating glaucoma or elevated intraocular pressure, wherein the method comprises administering an effective amount of a single composition comprising about 0.1% w/v brimonidine tartrate and about 0.68% w/v timolol maleate.
26. The method of item 25, wherein the composition is administered twice daily to an eye.
27.The method of item 26, wherein administration of the composition twice daily results in a lower incidence of one or more adverse events as compared to the twice-daily administration of a second single composition comprising a 0.2% w/v brimonidine tartrate and 0.5% w/v timolol maleate.
28.The method of item 27, wherein the one or more adverse events are selected from the group consisting of allergic conjunctivitis, conjunctival folliculosis, conjunctival hyperemia, eye pruritus, ocular burning, and ocular stinging.

## Claims

1. A composition comprising about 0.1% w/v brimonidine tartrate and about 0.68% w/v timolol maleate.

2. The composition of claim 1, wherein the composition comprises 0.1% w/v brimonidine tartrate and 0.68% w/v timolol maleate; preferably wherein:
i) the composition comprises 0.1% w/v brimonidine tartrate, 0.68% w/v timolol maleate, 0.1% w/v sodium chloride, 2.15% w/v sodium phosphate dibasic heptahydrate, 0.22% w/v sodium phosphate monobasic monohydrate, and water; or
ii) the composition comprises 0.1% w/v brimonidine tartrate, 0.68% w/v timolol maleate, 0.6% w/v boric acid, 0.38% sodium borate decahydrate, 0.5% w/v carboxymethyl cellulose, 0.32% w/v sodium chloride, and water.

3. The composition of claim 1, wherein the composition further comprises at least one buffer selected from the group consisting of sodium phosphate dibasic heptahydrate and sodium borate decahydrate.

4. The composition of any preceding claim, wherein:
i) the composition has a pH of about 7, preferably wherein the composition has a pH of 7.0; and/or
ii) the composition further comprises one or both of sodium hydroxide and hydrochloric acid; and/or
iii) administration of the composition twice daily is at least as effective as twice-daily administration of a second composition comprising a fixed dose of 0.2% w/v brimonidine tartrate and 0.5% w/v timolol maleate; and/or
iv) administration of the composition twice daily results in a lower incidence of one or more adverse events as compared to the twice-daily administration of a second composition comprising a fixed dose of 0.2% w/v brimonidine tartrate and 0.68% w/v timolol maleate, preferably wherein the one or more adverse events are selected from the group consisting of allergic conjunctivitis, conjunctival folliculosis, conjunctival hyperemia, eye pruritus, ocular burning, and ocular stinging.

5. The composition of any preceding claim, wherein:
i) the composition does not comprise a preservative; or
ii) the composition further comprises benzalkonium chloride, preferably wherein the benzalkonium chloride is present at a concentration of about 0.001% w/v to about 0.05% w/v.

6. The composition of any preceding claim, wherein:
i) the composition is configured for topical ocular administration; and/or
ii) the composition is for the treatment of glaucoma and elevated intraocular pressure.

7. A composition for reducing intraocular pressure, the composition consisting essentially of 0.1% w/v brimonidine tartrate, 0.68% w/v timolol maleate, 0.1% w/v sodium chloride, 2.15% w/v sodium phosphate dibasic heptahydrate, 0.22% w/v sodium phosphate monobasic monohydrate, and water.

8. A composition for reducing intraocular pressure, the composition consisting essentially of 0.1% w/v brimonidine tartrate, 0.68% w/v timolol maleate, 0.6% w/v boric acid, 0.38% sodium borate decahydrate, 0.5% w/v carboxymethyl cellulose, 0.32% w/v sodium chloride, and water.

9. An article of manufacture comprising packaging material and a pharmaceutical agent contained within the packaging material, wherein the packaging material comprises a label which indicates the pharmaceutical agent can be used for lowering intraocular pressure, and wherein the pharmaceutical agent is therapeutically effective for lowering intraocular pressure, the pharmaceutical agent comprising 0.1 % brimonidine tartrate and 0.68% timolol maleate.

10. The article of claim 9, wherein:
i) the pharmaceutical agent comprises 0.1% w/v brimonidine tartrate, 0.68% w/v timolol maleate, 0.1% w/v sodium chloride, 2.15% w/v sodium phosphate dibasic heptahydrate, 0.22% w/v sodium phosphate monobasic monohydrate, and water; or
ii) the pharmaceutical agent comprises 0.1% w/v brimonidine tartrate, 0.68% w/v timolol maleate, 0.6% w/v boric acid, 0.38% sodium borate decahydrate, 0.5% w/v carboxymethyl cellulose, 0.32% w/v sodium chloride, and water.

11. The article of any of claims 9-10, wherein the pharmaceutical agent is provided:
i) in a unit dose preservative-free configuration;
ii) in a multi dose preservative-free configuration; or
iii) in a multi dose configuration preserved with one or more preservative agents.

12. A method of treating glaucoma or elevated intraocular pressure, wherein the method comprises administering an effective amount of a single composition comprising about 0.1% w/v brimonidine tartrate and about 0.68% w/v timolol maleate.

13. The method of claim 12, wherein the composition is administered twice daily to an eye.

14. The method of claim 13, wherein administration of the composition twice daily results in a lower incidence of one or more adverse events as compared to the twice-daily administration of a second single composition comprising a 0.2% w/v brimonidine tartrate and 0.5% w/v timolol maleate.

15. The method of claim 14, wherein the one or more adverse events are selected from the group consisting of allergic conjunctivitis, conjunctival folliculosis, conjunctival hyperemia, eye pruritus, ocular burning, and ocular stinging.
